# EUROPÄISCHE PATENTSCHRIFT

(11) **EP 2 629 759 B1**
(45) Veröffentlichungstag und Bekanntmachung des Hinweises auf die Patenterteilung: **14.03.2018**
(21) Anmeldenummer: 11773411.1
(22) Anmeldetag: 20.10.2011
(51) Int. Cl.: A61K 9/16, A61K 9/50

(54) **VERBESSERTE PHARMAZEUTISCHE STARTERPELLETS**
IMPROVED PHARMACEUTICAL STARTER PELLETS
PELLET PHARMACEUTIQUES DE DÉPART AMÉLIORÉS

(30) Priorität: 22.10.2010 DE 102010050457
(43) Veröffentlichungstag der Anmeldung: 28.08.2013
(73) Patentinhaber: Südzucker Aktiengesellschaft Mannheim/Ochsenfurt, 68165 Mannheim (DE)
(72) Erfinder: BERNARD, Jörg, 67283 Albsheim (DE); LUHN, Oliver, 67134 Birkenheide (DE); KLEBOVICH, Imre, H-1145 Budapest (HU); ANTAL, Istvan, H-1165 Budapest (HU); KÁLLAI, Nikolett, 1174 Budapest (HU)
(74) Vertreter: Wohlfahrt, Jan Günther
(86) Internationale Anmeldenummer: PCT/EP2011/005297
(87) Internationale Veröffentlichungsnummer: WO 2012/052178

(56) Entgegenhaltungen:
- WO-A2-2007/090882
- NIKOLETT KÁLLAI ET AL: "Evaluation of Drug Release From Coated Pellets Based on Isomalt, Sugar, and Microcrystalline Cellulose Inert Cores", AAPS PHARMSCITECH, Bd. 11, Nr. 1, 1. März 2010 (2010-03-01), Seiten 383-391, XP55024443, ISSN: 1530-9932, DOI: 10.1208/s12249-010-9396-x
- BOWEN P: "Particle Size Distribution Measurement from Millimeters to Nanometers and from Rods to Platelets", JOURNAL OF DISPERSION SCIENCE AND TECHNOLOGY, TAYLOR AND FRANCIS GROUP, NEW YORK, NY, US, Bd. 23, Nr. 5, 1. Januar 2002 (2002-01-01) , Seiten 631-662, XP009102859, ISSN: 0193-2691, DOI: 10.1081/DIS-120015368

## Beschreibung

Die Erfindung betrifft verbesserte pharmazeutische Starterpellets aus einer Zusammensetzung, enthaltend ein Gemisch aus 1,6-GPS und 1,1-GPM, insbesondere Isomalt, und mikrokristalline Cellulose (MCC), sowie die Verwendung der Zusammensetzung in pharmazeutischen Starterpellets zur Verbesserung der Freisetzung eines pharmazeutischen Wirkstoffs aus den Pellets und zur Verbesserung der Verarbeitbarkeit.

Bei der oralen Verabreichung von Arzneimitteln soll der pharmazeutische Wirkstoff im Magen-Darm-Trakt kontrolliert freigesetzt und resorbiert werden. Zur pharmakodynamischen Steuerung der Freisetzung von Wirkstoffen von pharmazeutischen Trägern und der Resorption der Wirkstoffe sind bereits galenische Formulierungen bekannt. Eine Möglichkeit, diese Parameter zu steuern besteht darin, Darreichungsformen mit Überzügen zu versehen, die eine Wirkstofffreisetzung abhängig von der Löslichkeit oder Durchlässigkeit der Überzüge erlaubt. Damit ist auch eine kontrollierte Wirkstofffreisetzung möglich. Derartige Überzüge können beispielsweise auf Tabletten aufgebracht werden. Bei multipartikulären Formen, zum Beispiel Kapseln, ist die Gesamtmenge des Wirkstoffs auf eine größere Anzahl kleinerer Einheiten, sogenannte "Pellets", verteilt. Die Pellets werden jeweils mit Wirkstoff beladen und können zur kontrollierten Freisetzung des Wirkstoffs mit solchen Überzügen versehen sein. Ein Vorteil multipartikulärer Darreichungsformen ist, dass bei Beschichtung mit magensaftresistenten Überzügen die Freisetzung des Wirkstoffs in der Art gesteuert werden kann, dass sie über einen längeren Zeitraum erfolgt. Dies geschieht dadurch, dass sich die Pellets nach Einnahme im Magen verteilen und nur in Intervallen vom Magen in den Darmtrakt übertreten. Die Resorption des Wirkstoffs erfolgt in der Regel im Dünndarm.

Bekannte Substanzen, aus denen die noch nicht mit Wirkstoff beladenen oder unbeschichteten Pellets, als sogenannte Starterpellets, hergestellt sind, sind Saccharose (Sucrose) und Mannit, besonders aber mikrokristalline Cellulose (MCC). Starterpellets auf Saccharosebasis zeichnen sich durch gute Wirkstofffreisetzung im Körper aus, da das Trägermaterial gut wasserlöslich ist bzw. schnell zerfällt. Saccharosepellets weisen jedoch eine schlechte Verarbeitbarkeit, zum Beispiel eine hohe Friabilität und einen damit verbundenen hohen Abrieb auf. Bei Saccharosepellets kann die Beschichtung mit Wirkstoff aus wässrigen Sprühlösungen oder Sprühsuspensionen zu Beginn des Beschichtungsprozesses nur mit relativ niedrigen Sprühraten durchgeführt werden, da es durch das Anlösen der Oberfläche zu Auflösungserscheinungen oder Verklumpungen kommt. Dies bedingt eine nachteilig lange Verweilzeit der Pellets in der Wirbelschicht. Starterpellets aus MCC zeichnen sich durch eine gute Verarbeitbarkeit aus. Sie weisen eine geringe Friabilität, d. h. einen geringen Abrieb auf und ermöglichen so zu Beginn des Beschichtungssprozesses vergleichsweise höhere Sprühraten, woraus verringerte Prozessverweilzeiten folgen. MCC-Matrix-Pellets können den pharmazeutischen Wirkstoff jedoch weitaus schlechter freisetzen, da sie nicht wasserlöslich sind und im Körper nicht enzymatisch abgebaut werden. In Kontakt mit Wasser oder Verdauungssäften quellen die aus MCC bestehenden Pellets auf und der Wirkstoff bleibt teilweise an die MCC-Fasern adsorbiert.

Die Osmolalität als Maß der osmotischen Bedingungen im Magen-Darm-Trakt schwankt je nach Befüllung und aufgenommener Nahrung zwischen 100 und 1000 mOsm/kg, insbesondere zwischen 100 und 400 mOsm/kg. In mit einer wasserunlöslichen und permeablen Membran beschichteten Pellets, die sich im Magen-Darm-Trakt befinden, baut sich durch einströmendes Wasser ein interner osmotischer Druck zwischen inneren und äußeren Pelletschichten auf, sofern eine osmotisch aktive Substanz innerhalb der Membran vorliegt. Die Freisetzung des Wirkstoffs wird unterstützt durch den osmotischen Gradienten. Eine hohe Osmolalität des Umgebungsmediums führt bei bekannten Pellets aus MCC zu einem geringeren osmotischen Gradienten und einer verlangsamten Freisetzung des Wirkstoffs. Der Zeitpunkt der Einnahme eines Medikaments muss daher mit den osmotischen Bedingungen im Magen-Darm-Trakt, besonders also mit der Einnahme einer Mahlzeit, abgestimmt werden. Alle bisher bekannten Starterpellets sind, vor allem im Hinblick auf die Verarbeitung und Einsetzbarkeit bei der pharmazeutischen Behandlung, verbesserungswürdig.

WO 2007/090882 A2 offenbart pharmazeutische Zusammensetzungen für eine verzögerte Wirkstofffreisetzung, die in besonderen Ausführungsformen mikrokristalline Cellulose (MCC) und Zuckeralkohole enthalten können.

Kállai et al. (Evaluation of Drug Release From Coated Pellets Based on Isomalt, Sugar, and Microcrystalline Cellulose Inert Cores, AAPS PharmSciTech, 2010, Vol. 11, No. 1, 383-391) untersucht die Effekte der Verwendung von Isomalt, Zucker oder mikrokristalliner Cellulose in pharmazeutischen Zusammensetzungen auf die Freisetzungskinetik von Wirkstoffen.

Ein der vorliegenden Erfindung zugrundeliegendes technisches Problem besteht darin, ein pharmazeutisches Starterpellet bereitzustellen worin die Wirkstofffreisetzung weitgehend unabhängig von der Osmolalität des Umgebungsmediums ist. Ein weiteres technisches Problem besteht darin, ein pharmazeutisches Starterpellet bereitzustellen, das eine effizientere Beschichtung und bessere Verarbeitung erlaubt.

Diese technischen Probleme werden gelöst durch den Gegenstand der Ansprüche, insbesondere durch ein pharmazeutisches Starterpellet aus einer Zusammensetzung, enthaltend als erste Komponente a) ein Gemisch aus 6-O-α-D-glucopyranosyl-D-sorbit (1,6-GPS) und 1-O-α-D-glucopyranosyl-D-mannit (1,1-GPM), insbesondere Isomalt, und als zweite Komponente b) mikrokristalline Cellulose (MCC). Das Starterpellet enthält diese Zusammensetzung in einem wesentlichen Anteil oder besteht daraus.

Pellets aus dieser Zusammensetzung setzen einen pharmazeutischen Wirkstoff, mit dem sie beschichtet sind, vollständiger und schneller frei als bekannte Pellets aus mikrokristalliner Cellulose. Die erfindungsgemäßen Pellets auf Basis der erfindungsgemäßen Zusammensetzung können einen pharmazeutischen Wirkstoff, mit dem sie beschichtet sind, weitgehend oder vollständig unabhängig von der Osmolalität des Umgebungsmediums freisetzen. Dies ist vor allem bei selbst schlecht löslichen Wirkstoffen wie Diclofenac-Na zu beobachten.

Eine Beimengung von bereits 10 % der Komponente a) in den erfindungsgemäßen pharmazeutischen Starterpellets führt zu einer signifikant verbesserten Freisetzung des pharmazeutischen Wirkstoffs und zu einem signifikant geringeren Einfluss der Osmolalität des Umgebungsmediums als bei auf MCC-Starterpellets basierenden Formulierungen. Trotz eines überwiegenden Gehalts MCC, insbesondere einem Gehalt von mehr als 50 % des Gesamtpellets, tritt der bekannte nachteilige Effekt von MCC-Pellets auf die Freisetzung des pharmazeutischen Wirkstoffs überraschenderweise in den Hintergrund.

Ohne an die Theorie gebunden sein zu wollen, wird durch die Beimengung der löslichen, osmotisch aktiven ersten Komponente a) in die Grundsubstanz des Starterpellets eine erhöhte Löslichkeit vermittelt, die zu der deutlichen Verbesserung der Freisetzung des Wirkstoffs führt. Erfindungsgemäß beträgt der Anteil der Komponente a) in der Zusammensetzung für das Starterpellet 1 Gew.-% oder mehr, besonders 5 Gew.-% oder mehr, oder 10 Gew.-% oder mehr (jeweils bezogen auf die Trockensubstanz). In besonderen Varianten davon beträgt der Anteil der Komponente a) in der Zusammensetzung maximal 60 Gew.-%, besonders maximal 50 Gew.-% oder maximal 40 Gew.-% oder maximal 30 Gew.-%.

In einer Ausführung der Erfindung liegen die Komponenten a) und b) in der Zusammensetzung für das Starterpellet in einem Verhältnis von 1:99 bis 60:40 Gew.-% (bezogen auf die Trockensubstanz) vor. In einer besonderen Ausführung der Erfindung liegen die Komponenten a) und b) in der Zusammensetzung in einem Verhältnis von 5:95 bis 60:40 Gew.-% (bezogen auf die Trockensubstanz) vor. In einer besonderen Ausführung der Erfindung liegen die Komponenten a) und b) in der Zusammensetzung in einem Verhältnis von 10:90 bis 50:50 Gew.-% (bezogen auf die Trockensubstanz) vor.

In einer speziellen Variante liegen die Komponenten a) und b) in der Zusammensetzung in einem Verhältnis von etwa 50:50 Gew.-% (bezogen auf die Trockensubstanz) vor. In einer anderen speziellen Variante liegen die Komponenten a) und b) in der Zusammensetzung in einem Verhältnis von etwa 30:70 Gew.-% (bezogen auf die Trockensubstanz) vor. In einer anderen speziellen Variante liegen die Komponenten a) und b) in der Zusammensetzung in einem Verhältnis von 10:90 Gew.-% (bezogen auf die Trockensubstanz) vor.

In einer Ausführung der Erfindung ist die Komponente a) des pharmazeutischen Starterpellets ein Gemisch enthaltend 1 bis 99 Gew.-% 1,6-GPS und 99 bis 1 Gew.-% 1,1-GPM (bezogen auf die Trockensubstanz).

In einer besonders bevorzugten Ausführungsform der Erfindung ist die Komponente a) des pharmazeutischen Starterpellets ein Gemisch enthaltend 47 bis 53 Gew.-% 1,6-GPS und 53 bis 47 Gew.-% 1,1-GPM (bezogen auf die Trockensubstanz). Diese Disaccharidalkohol-Zusammensetzung wird allgemein auch als Isomalt, Palatinit® und galenIQ™ bezeichnet.

In einer besonders bevorzugten Ausführungsform ist die Komponente a) der Zusammensetzung für das pharmazeutische Starterpellet ein Gemisch enthaltend 20 bis 48 Gew.-% 1,6-GPS und 80 bis 52 Gew.-% 1,1-GPM, besonders 20 bis 38 Gew.-% 1,6 GPS und 80 bis 62 Gew.-% 1,1 GPM (bezogen auf die Trockensubstanz), das heißt eine gegenüber dem rein racemischen Gemisch von 1,6-GPS und 1,1-GPM mit 1,1-GPM angereicherte Variante des Isomalts.

Bevorzugt besteht die Komponente a) aus gemahlenen Bestandteilen. Komponente a) weist besonders eine Partikelgrößenverteilung von 9-22-41 µm (d10-d50-d90 Quantilen, durch Laserbeugung gemessen) auf. Bevorzugt eingesetzte Mischungen sind die gemahlenen Isomaltvarianten galenIQ® 800 (Fa. Beneo-Palatinit, DE; Isomalt Ph. Eur., BP, USP29-NF24) oder ähnliche Produkte.

Vorteilhafterweise sind die erfindungsgemäßen Starterpellets also zuckerfrei und somit für Diabetiker besser geeignet als auf Saccharose (Sucrose) basierende Pellets.

Außerdem sind die erfindungsgemäßen Starterpellets akariogen und so besser für orale Applikationen, besonders zur Wirkstofffreisetzung in Mund und Rachen, geeignet.

Erfindungsgemäße Starterpellets können mittels üblicher Methoden aus der erfindungsgemäßen Zusammensetzung hergestellt werden. In einer Ausgestaltung erfolgt die Herstellung in an sich bekannter Weise durch Extrusion und anschließende Sphäronisation des Extrudats. Die Trockenmischung aus der erfindungsgemäßen Zusammensetzung wird dazu in einem Pflugscharmischer gemischt und nachfolgend durch Zugabe einer Flüssigkeit, bevorzugt Wasser, granuliert. Die Extrusion der granulierten Masse in einem Extruder erfolgt besonders mit einem Durchmesser der Düsenplatte von 500 bis 1500 µm. Die Mischung der Komponenten mit Wasser kann jedoch ebenfalls im Extruder selbst erfolgen, wobei die Zusammenstellung der Schneckengeometrien, besonders das Verhältnis von Förder-, Misch- und Knetelementen, zur Sicherstellung einer homogenen Verteilung des Wassers und eines gewünschten Verdichtungsgrades, rezepturabhängig eingestellt wird. Die Sphäronisation der extrudierten Bruchstücke erfolgt in einem Sphäronizer in an sich bekannter Weise. Gegebenenfalls werden die erhaltenen verrundeten Pellets in der Wirbelschicht getrocknet, wobei bekanntermaßen der Durchmesser schrumpft. Mittels eines Taumelsiebs mit mehreren Siebeinsätzen zwischen 100 bis 1500 µm, oder 200 bis 1000 µm Durchmesser, besonders 300 bis 800 µm Durchmesser, werden die Starterpellets sortiert. Eine Sortierung nach Rundheit kann durch das Nachschalten von Sortiertischen erfolgen, die nach dem Prinzip der Massenträgheit arbeiten.

Für die Beschichtung von pharmazeutischen Starterpellets ist es vorteilhaft, wenn die Starterpellets möglichst kugelförmig vorliegen. Als kugelförmig wird ein Pellet dann bezeichnet, wenn das Verhältnis aus dem Quadrat des Perimeters (Umfang) und 4 * Pi * Projektionsfläche weniger als 1,24 beträgt. Die Starterpellets gemäß der Erfindung weisen vorteilhafterweise eine durchschnittliche Sphärizität von maximal 1,06, besonders von maximal 1,05, vor allem von maximal 1,04 auf. Die Starterpellets gemäß der Erfindung weisen besonders einen Durchmesser von 0,2 bis 2,0 mm oder 0,6 bis 1,2 mm, besonders von 0,3 bis 0,6 mm auf.

Die erfindungsgemäßen Starterpellets weisen eine glatte Oberfläche auf. Dies erlaubt vorteilhafterweise eine effiziente und kontrollierte Beschichtung bzw. Beladung der Starterpellets, wobei sowohl Beschichtungsmaterial als auch Prozessverweilzeit eingespart werden können. Gleichzeitig kann eine gleichmäßigere Schichtdicke erreicht werden, was die Kontrollierbarkeit der Wirkstofffreisetzung begünstigt.

Vorteilhafterweise neigen die erfindungsgemäßen Starterpellets während der Beschichtung bzw. Beladung in der Wirbelschicht deutlich weniger zu Agglomeration oder sogenannter Zwillingsbildung als bekannte auf Saccharose basierte oder reine MCC-Starterpellets.

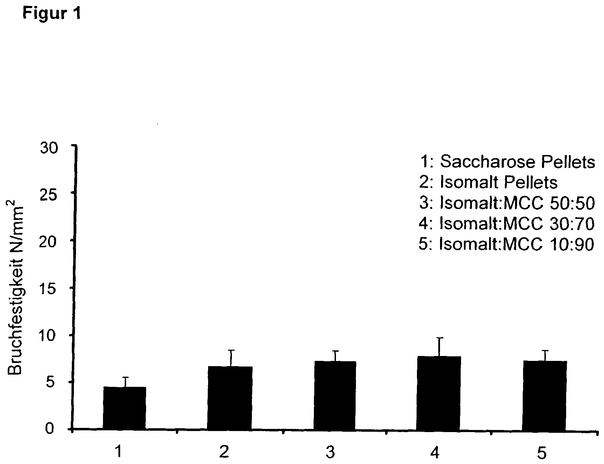

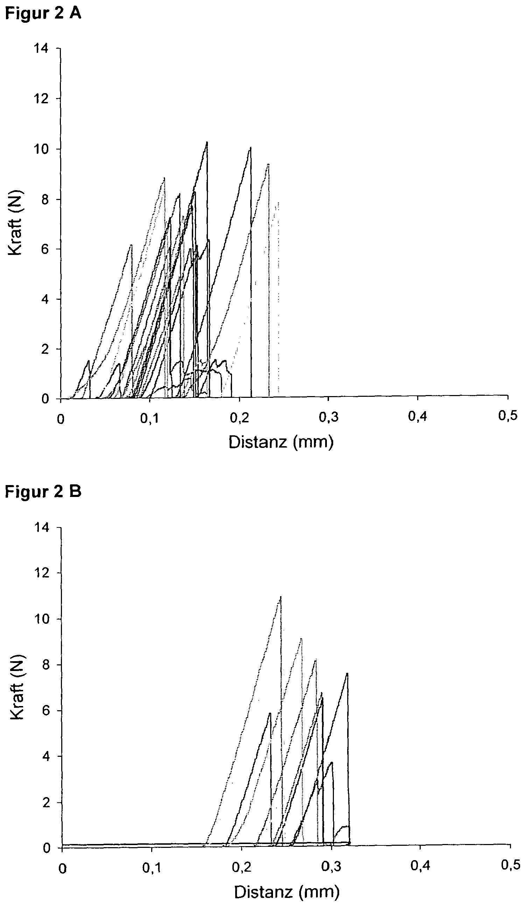

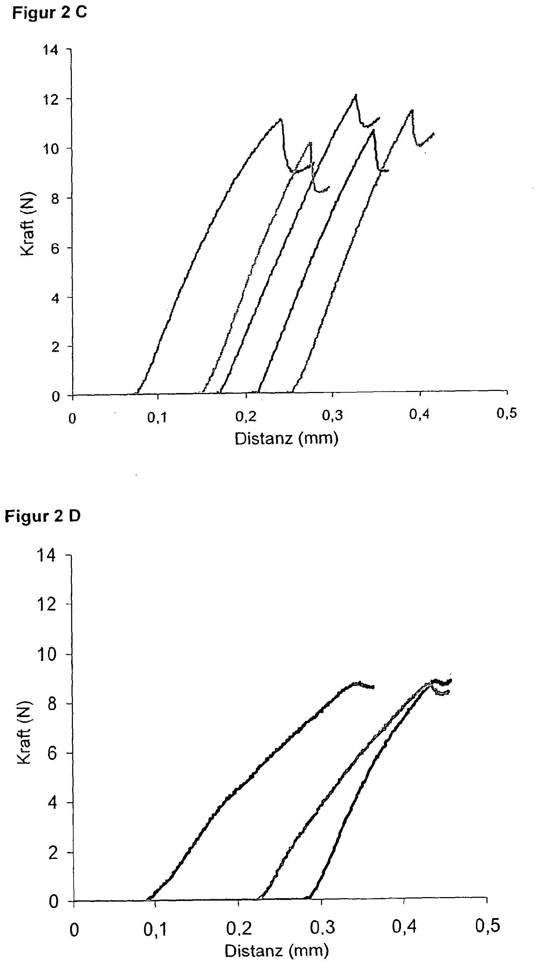

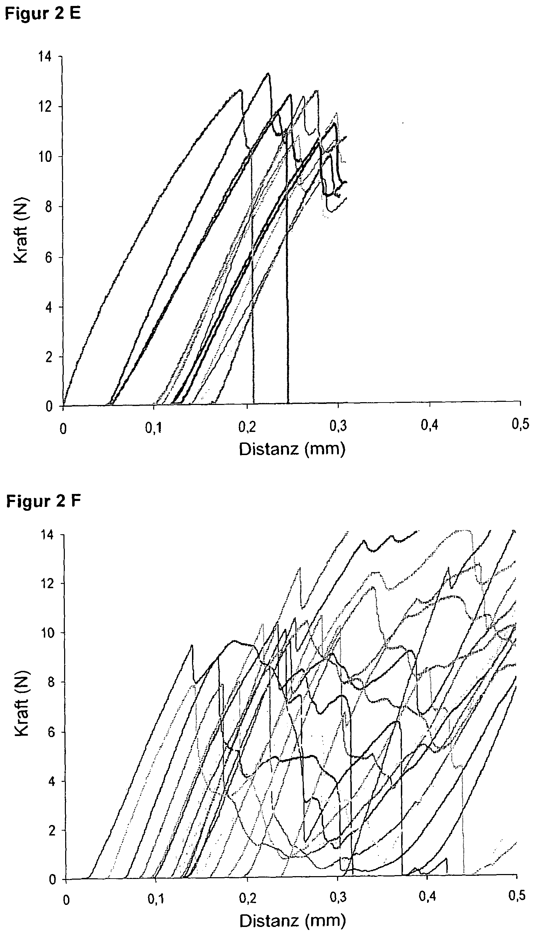

Die Bruchfestigkeit (N/mm²) der erfindungsgemäßen Starterpellets entspricht mit ca. 6 bis 8 in etwa derjenigen von Starterpellets aus purem Isomalt (siehe Figur 1). Eine Untersuchung der Kraft-Weg-Kurven, die das Verformungsverhalten der Pellets widerspiegeln, zeigt, dass Starterpellets aus Saccharose (Vergleich) ebenso wie Pellets aus purem Isomalt (Vergleich) sehr spröde sind und daher eine hohe Friabilität, also einen nachteilig hohen Abrieb bei der Verarbeitung aufweisen (siehe Figur 2A, B). Die erfindungsgemäßen Starterpellets hingegen zeigen vorteilhafterweise ein duktiles oder plastisches Verhalten, was zu geringer Friabilität und somit zu einer deutlich besseren Verarbeitbarkeit führt (siehe Figur 2C, D, E), so dass insbesondere während der Verarbeitung in der Wirbelschicht kein oder zumindest wenig Abrieb entsteht. Erfindungsgemäße Starterpellets mit einer Zusammensetzung der Komponenten a) und b) im Verhältnis von 30:70 Gew.-% weisen dabei das am meisten plastische Verhalten verglichen mit erfindungsgemäßen Starterpellets einer Zusammensetzung von 50:50 Gew.-% oder 10:90 Gew.-% auf.

In einer Ausgestaltung sind die Starterpellets also mit mindestens einem pharmazeutischen Wirkstoff beschichtet und zwar in Form einer Schicht an deren Oberfläche. Alternativ oder zusätzlich ist vorgesehen, den mindestens einen oder weiteren pharmazeutischen Wirkstoff bereits in die Zusammensetzung der Starterpellets einzubringen und somit direkt Pellets zu erhalten, die den Wirkstoff im Grundmaterial enthalten.

Vorliegend ist unter dem Begriff "pharmazeutischer Wirkstoff" eine Substanz oder ein Agens zu verstehen, das einen physiologischen und/oder psychologischen Effekt in einem menschlichen oder tierischen Körper auslöst und vornehmlich zur Behandlung, Vorbeugung oder Diagnose von Krankheiten bei Mensch oder Tier dient. Darunter zählen auch Wirkstoffe, die ihre Wirkung an in den Körper eingedrungenen Krankheitserregern wie Bakterien, Pilze, Parasiten und Viren ausüben können. Weiter zählen dazu auch inerte Substanzen wie Kontrastmittel, Adsorbantien sowie Mittel zur Prävention oder Behandlung chemischer oder radioaktiver Belastung.

Weiter ist vorliegend unter dem Begriff "pharmazeutischer Wirkstoff" auch ein Nahrungsergänzungsmittel, Prä- oder Probiotikum, Vitaminpräparat, omega 3- und omega 6-Fettsäuren (DHA, EPA, GLA) oder eine kosmetisch wirksame Substanz zu verstehen, die dem menschlichen oder tierischen Körper zugeführt werden soll.

In einer besonderen Ausgestaltung sind oder werden die Pellets mit einem schwer wasserlöslichen pharmazeutischen Wirkstoff beschichtet, insbesondere einem Wirkstoff der eine Löslichkeit in Wasser (25° C) von 50 mg/ml oder weniger, besonders von 10 mg/ml oder weniger oder von 5 mg/ml oder weniger aufweist. Ein solcher pharmazeutischer Wirkstoff ist beispielweise Diclofenac NatriumSalz, das eine Löslichkeit von 2,4 mg/ml (25° C) aufweist. Bevorzugt wird eine Wirkstoffbeschichtung von 3 bis 50 Gew.-% bezogen auf das Trockengewicht des Starterpellets erreicht.

In einer erfindungsgemäßen Ausgestaltung ist der pharmazeutische Wirkstoff mit Zusatzstoffen, wie z. B. Bindemitteln, auf die pharmazeutischen Starterpellets aufgebracht. Bevorzugt ist als Bindemittel Hydroxypropylmethylcellulose (HPMC). Andere Bindemittel sind ebenfalls möglich.

Die erfindungsgemäßen pharmazeutischen Starterpellets sind bevorzugt zusätzlich mit einer Schicht, insbesondere einem Film oder Überzug, zur kontrollierten Freisetzung des pharmazeutischen Wirkstoffs beschichtet. Derartig behandelte Pellets bestehen primär aus einem Kern, das heißt dem Starterpellet, mindestens einer Wirkstoffschicht und mindestens einer Beschichtung zur kontrollierten Freisetzung des oder der Wirkstoffe. Als Beschichtungen zur kontrollierten Freisetzung von pharmazeutischen Wirkstoffen sind vorzugsweise 20 bis 100 µm dicke polymerbasierte Filme oder Überzüge vorgesehen. Hierzu ist die polymere Substanz in Wasser oder einem organischen Lösungsmittel gelöst oder suspendiert und wird auf die vorzugsweise in der Wirbelschicht fluidisierten Pellets aufgesprüht. Bevorzugt erfolgt die Beschichtung in der Wirbelschicht mit Sprüheinsatz am Boden und einem Rohr zur Vereinheitlichung der Verweilzeit der Pellets (z. B. Wurster-Einsatz). Das Auftragen der Wirkstoffbeschichtung und der Schicht zur kontrollierten Freisetzung kann auch durch andere übliche Verfahren erfolgen.

Die eingesetzten Beschichtungen zur kontrollierten Freisetzung unterscheiden sich beispielsweise in der Permeabilität, die weiterhin durch verschiedene Zusätze wie quellbare Polymere spezifisch angepasst werden kann. Durch die Wahl der funktionellen Gruppen der eingesetzten Polymere kann beispielsweise auch eine pH-Wert Abhängigkeit eingestellt werden, um eine Freisetzung des pharmazeutischen Wirkstoffs in verschiedenen Abschnitten des Magen-Darm-Trakts zu steuern. Enterische Beschichtungen verhindern eine Freisetzung im Magen, sind also gegenüber einem sauren pH-Wert resistent und sorgen so für eine spätere Freisetzung des Wirkstoffs im Darmtrakt. Andere Beschichtungskomponenten bilden Poren in einem ansonsten schwer löslichen Überzug und sorgen so für eine langsame, länger anhaltende Freisetzung des Wirkstoffs.

Zur Beschichtung der erfindungsgemäßen Starterpellets können alle bekannten, pharmazeutisch akzeptablen polymeren Filmbildner, ggf. unter Beimengung eines Verflüssigers wie z. B. Triethylcitrat, eingesetzt werden. Besonders bevorzugt werden die erfindungsgemäßen Starterpellets mit Polymethacrylat-Kopolymeren beschichtet, vorzugsweise Eudragit® RS oder RL. Bevorzugt beträgt die Eudragit® RS oder RL Schicht 5 bis 10 Gew.-% bezogen auf das Pelletgewicht. Weiterhin können in die Schicht zur kontrollierten Freisetzung auch weitere Hilfsstoffe eingebracht werden, wie beispielsweise Pigmente, Lösungsmittel, Trübungsmittel, Gleitmittel wie z. B. Talk, Geschmacksstoffe, Substanzen zur Geschmacksmaskierung oder ähnliche. Die Schicht zur kontrollierten Freisetzung wird in einer Zusammensetzung und einer ausreichenden Dicke auf die erfindungsgemäßen Starterpellets aufgetragen, die das erwünschte Freisetzungsprofil ergibt. Hierbei muss darauf geachtet werden, dass die Schichtdicke eine mechanische Stabilität des Films gewährleistet, so dass ein Bruch der Schicht und die damit einhergehende unkontrollierte Freisetzung des Wirkstoffs vermieden wird.

Bevorzugt werden die mit pharmazeutischem Wirkstoff beladenen und ggf. einem Film zur kontrollierten Freisetzung beschichteten pharmazeutischen Starterpellets zur Verabreichung in Hartgelatinekapseln gefüllt. Es kann auch vorgesehen sein, die mit pharmazeutischem Wirkstoff beladenen und ggf. einem Film zur kontrollierten Freisetzung beschichteten erfindungsgemäßen Starterpellets in Mischungen mit anderen Hilfsstoffen zu Tabletten zu verpressen. In beiden Fällen können auch jeweils mit verschiedenen pharmazeutischen Wirkstoffen beschichtete Starterpellets, die beispielsweise nicht innerhalb einer Formulierung kompatibel sind, in einer Kapsel oder Tablette gemischt werden, um zwei oder mehrere Wirkstoffe gemeinsam zu verabreichen. Des Weiteren kann es auch vorgesehen sein, dass die mit jeweils unterschiedlichen Wirkstoffen beladenen erfindungsgemäßen Starterpellets mit jeweils unterschiedlichen Filmen oder Überzügen beschichtet sind, so dass die verschiedenen Wirkstoffe beispielsweise an unterschiedlichen Orten des Magen-Darm-Trakts oder zu unterschiedlichen Zeitpunkten freigesetzt werden können.

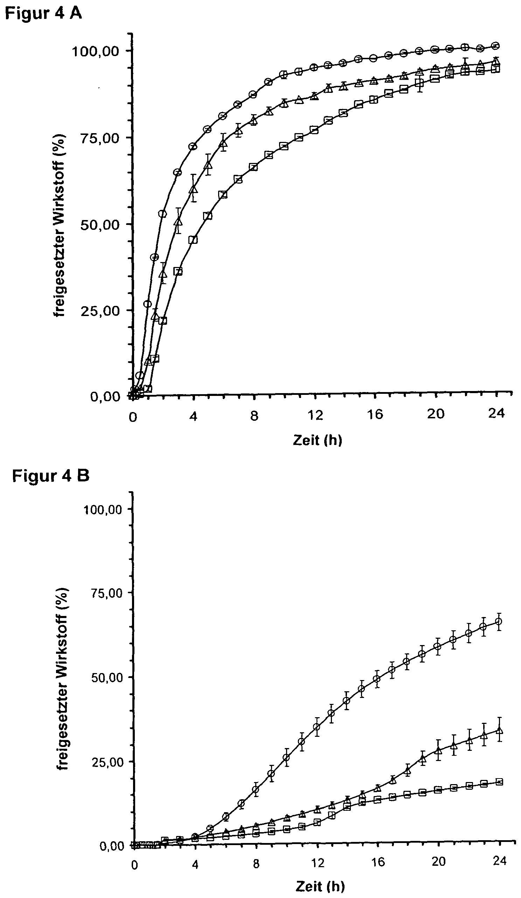

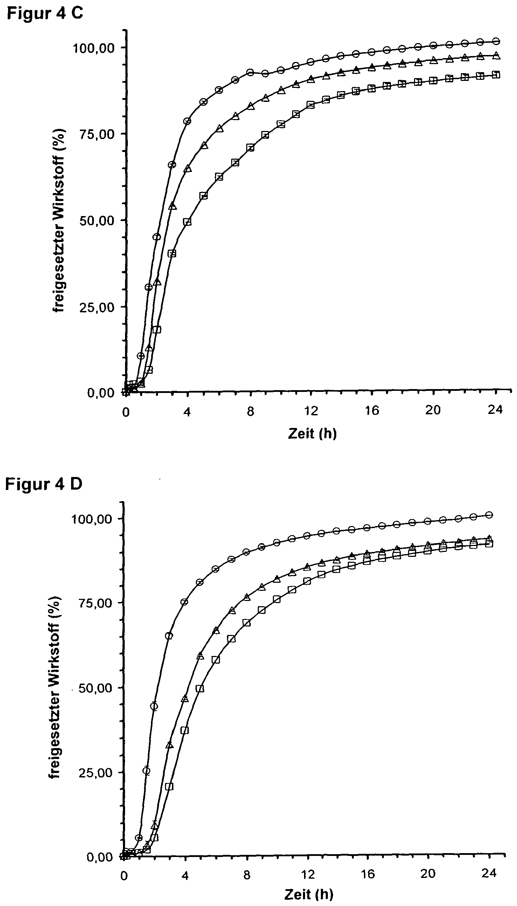

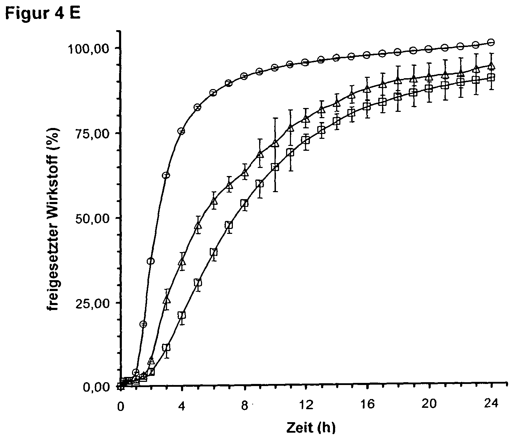

Gegenstand der Erfindung ist auch die Verwendung der erfindungsgemäßen Zusammensetzung der Komponenten a) und b) als Bestandteil von pharmazeutischen Starterpellets zum Zwecke der Verbesserung der Freisetzung eines pharmazeutischen Wirkstoffs aus dem Pellet. Besonders ist die verbesserte Freisetzung des pharmazeutischen Wirkstoffs dadurch gekennzeichnet, dass sie weitgehend oder völlig unabhängig von der Osmolalität des Umgebungsmediums ist. Es konnte beispielsweise gezeigt werden, dass der Wirkstoff Diclofenac-Na aus MCC-Starterpellets mit 5 % Eudragit®-Beschichtung bei normalen osmotischen Bedingungen nach 16 h zu ca. 48 % freigesetzt wurde, bei einer Osmolalität von 0,483 Osm/kg jedoch nur zu ca. 16 % und bei einer Osmolalität von 0,706 Osm/kg nur zu ca. 12 % (siehe Figur 4B). Das heißt, durch die Erhöhung der Osmolalität des Umgebungsmediums wurde die Freisetzung des Wirkstoffs von ca. 48 % auf ca. 16 % bzw. ca. 12 % reduziert. Aus den erfindungsgemäßen Starterpellets mit einer Zusammensetzung von Komponente a) und b) im Verhältnis von 50:50 Gew.-% und 5 % Eudragit®-Beschichtung war der Wirkstoff Diclofenac-Na unter normalen osmotischen Bedingungen nach 16 h bereits zu ca. 97 % freigesetzt (siehe Figur 4C). Durch eine erhöhte Osmolalität (0,483 Osm/kg) wurde die Freisetzung kaum beeinträchtigt, da nach 16 h zumindest ca. 94 % des Wirkstoffs freigesetzt waren. Auch eine weitere Erhöhung der Osmolalität (0,706 Osm/kg) reduzierte die Freisetzung nur auf ca. 86 %. Für die erfindungsgemäßen Starterpellets mit einer Zusammensetzung von Komponente a) und b) im Verhältnis von 30:70 Gew.-%, bzw. 10:90 Gew.-% und 5 % Eudragit®-Beschichtung gilt dies ebenfalls: hier konnte eine Freisetzung unter normalen osmotischen Bedingungen nach 16 h von ca. 97 % bzw. ca. 96 % des Wirkstoffs festgestellt werden (siehe Figur 4D und E). Eine erste Erhöhung der Osmolalität auf 0,483 Osm/kg führte zu einer Freisetzung von ca. 89 % bzw. ca. 87 % des Wirkstoffs nach 16 h. Bei einer Osmolalität von 0,706 Osm/kg konnte dagegen immer noch eine Freisetzung von ca. 86 % bzw. ca. 82 % beobachtet werden. Die Freisetzung des Wirkstoffs aus den erfindungsgemäßen Starterpellets erfolgt somit zum einen schneller als aus MCC-Starterpellets und zum anderen weitgehend unabhängig von der Osmolalität des Umgebungsmediums.

Gegenstand der Erfindung ist auch die Verwendung der erfindungsgemäßen Zusammensetzung als Bestandteil von pharmazeutischen Starterpellets zum Zwecke der Verringerung des Abriebs bei der Verarbeitung der Pellets.

Ein weiterer Gegenstand der Erfindung ist auch die Verwendung der erfindungsgemäßen Zusammensetzung als Bestandteil von pharmazeutischen Starterpellets zum Zwecke der Verbesserung der kontrollierten Beschichtung des Starterpellets mit einem pharmazeutischen Wirkstoff und/oder einem Überzug zur kontrollierten Freisetzung des Wirkstoffs, besonders zur Verringerung der Verweilzeit in der Wirbelschicht während der Beschichtung mit Wirkstoff und/oder dem Überzug, da höhere Sprühraten eingesetzt werden können. Außerdem kann eine gleichmäßigere Schichtdicke bei gleichzeitiger Einsparung von Beschichtungsmaterial erzielt werden. Überraschenderweise zeigen die erfindungsgemäßen Starterpellets bereits bei einer Beimengung von etwa 50 Gew.-% MCC nicht mehr die Auflösungs- bzw. Verklumpungserscheinungen während der Beschichtung in der Wirbelschicht, d. h. sie zeigen ein den MCC-Pellets ähnlicheres Verhalten.

Weitere bevorzugte Ausführungsformen der vorliegenden Erfindung ergeben sich auch aus den Merkmalen der Unteransprüche.

Die vorliegende Erfindung wird anhand der Figuren und Beispiele näher erläutert.

Figur 1 zeigt die Bruchfestigkeit in N/mm² von pharmazeutischen Pellets auf Saccharosebasis (1), aus purem Isomalt (2) und der erfindungsgemäßen Zusammensetzung (Isomalt:MCC 50:50 (3), 30:70 (4) und 10:90 (5) Gew.-%).

Figuren 2A bis F zeigen als Kraft-Weg-Kurven das Verformungsverhalten von pharmazeutischen Pellets auf Saccharosebasis (A), purem Isomalt (B), Isomalt:MCC 50:50 Gew.-% (C), 30:70 Gew.-% (D), 10:90 Gew.-% (E) und MCC (F).

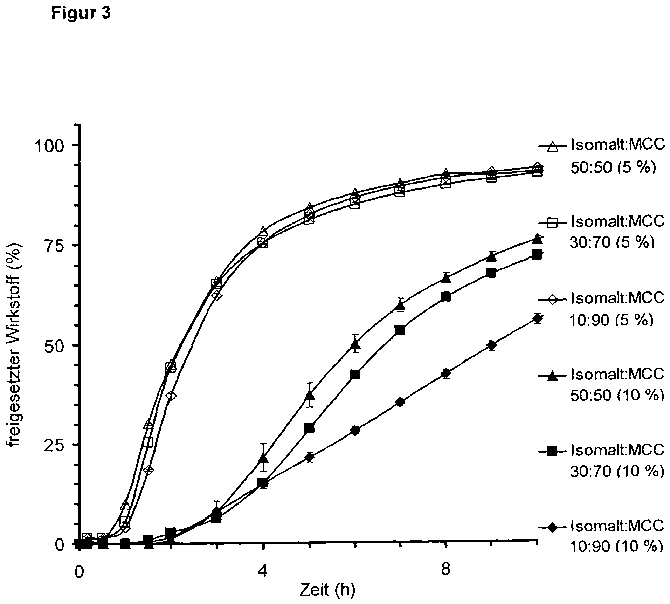

Figur 3 zeigt die Freisetzung des Wirkstoffs Diclofenac-Na aus den erfindungsgemäßen pharmazeutischen Pellets aus Isomalt:MCC 50:50 (Linie mit Dreieck), 30:70 (Linie mit Quadrat) und 10:90 (Linie mit Raute) Gew.-% jeweils mit 5 % Eudragit® Beschichtung (Linien mit leeren Symbolen) oder mit 10 % Eudragit® Beschichtung (Linien mit gefüllten Symbolen).

Figuren 4A bis E zeigen die Freisetzung des Wirkstoffs Diclofenac-Na aus pharmazeutischen Pellets aus purem Isomalt (A), MCC (B), Isomalt:MCC 50:50 (C), 30:70 (D) und 10:90 (E) Gew.-% mit jeweils 5 % Eudragit® Beschichtung bei normaler Osmolalität (0,106 Osm/kg, Linie mit Kreis) oder bei erhöhten Osmolalitäten (0,483 Osm/kg, Linie mit Dreieck; 0,706 Osm/kg, Linie mit Quadrat). Die Erhöhung der Osmolalität wurde durch Zugabe von 0,25 mol/l Glukoseanhydrat bzw. 0,5 mol/l Glukoseanhydrat zum Medium erreicht.

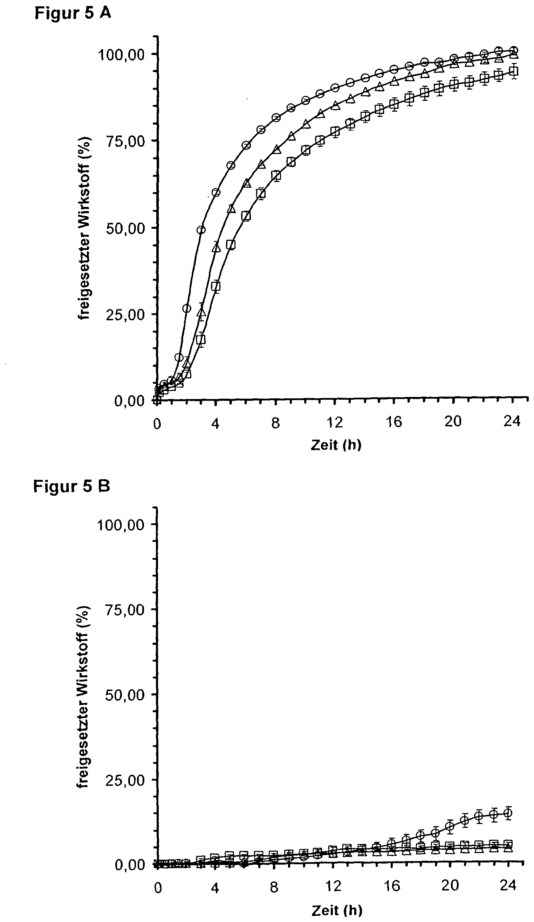

Figuren 5A bis E zeigen die Freisetzung des Wirkstoffs Diclofenac-Na aus pharmazeutischen Pellets aus purem Isomalt (A), MCC (B), Isomalt:MCC 50:50 (C), 30:70 (D) und 10:90 (E) Gew.-% mit jeweils 10 % Eudragit® Beschichtung bei normaler Osmolalität (0,106 Osm/kg, Linie mit Kreis) oder bei erhöhten Osmolalitäten (0,483 Osm/kg, Linie mit Dreieck; 0,706 Osm/kg, Linie mit Quadrat). Die Erhöhung der Osmolalität wurde durch Zugabe von 0,25 mol/l Glukoseanhydrat bzw. 0,5 mol/l Glukoseanhydrat zum Medium erreicht.

### Beispiel 1: Herstellung der pharmazeutischen Starterpellets

Tabelle 1 zeigt die Rezepturen der pharmazeutischen Starterpellets.

**Tabelle 1:**

| **MCC-Anteil [%]** | **Isomalt**-**Anteil [%]** | **Wassergehalt [%]** |
|---|---|---|
| 90 | 10 | 94 |
| 70 | 30 | 60 |
| 60 | 40 | 55 |
| 50 | 50 | 40 |

Wassergehalt bezogen auf die Trockensubstanz.

### Herstellungsverfahren

Trockenmischung: Anhand der in Tabelle 1 aufgeführten prozentualen Anteile wurde jeweils eine 5 kg Zusammensetzung aus MCC und Isomalt zunächst ohne den Wasseranteil in einem Pflugscharmischer (M20, Fa. Lödige) mit einer Drehzahl von 106 min⁻¹ für 3 Minuten gemischt.

Granulierung: Anschließend wurde die entsprechende Menge Wasser (dest.) zugegeben und die Mischung ebenfalls mit einer Drehzahl von 106 min⁻¹ für 3 Minuten granuliert.

Extrusion: Die granulierte Zusammensetzung wurde in einen Extruder (DE-40-T-10D, Fa. Gabler) gegeben und mit einer Drehzahl von 60 min⁻¹ entweder durch Düsenplatten mit 500 µm oder 1000 µm Durchmesser extrudiert.

Sphäronisation: Extrudierte Bruchstücke wurden in einem Sphäronizer (R-600, Fa. Gabler) bei einer Drehzahl von 550 1/min verrundet.

Trocknung: Die verrundeten Starterpellets wurden für 2,5 bis 3 Stunden bei einer Zulufttemperatur von 100 bis 50° C in einem Wirbelschichttrockner (Aeromatic Strea 7, Fa. Aeromatic) getrocknet.

Klassierung: Die getrockneten Starterpellets wurden über ein Taumelsieb mit 700 µm und 1000 µm Siebeinsätzen klassiert.

Tabelle 2 zeigt den Gesamtwassergehalt der getrockneten Starterpellets gemessen nach Karl Fischer.

**Tabelle 2:**

| **Massenanteil** | **Wassergehalt [%]** |
|---|---|
| 90 % MCC + 10 % Isomalt | 5,8 |
| 70 % MCC + 30 % Isomalt | 5,8 |
| 50 % MCC + 50 % Isomalt | 5,8 |

### Beispiel 2: Wirkstoffbeschichtung der pharmazeutischen Starterpellets

### Herstellung der Lösung

HPMC wurde in ein Drittel des Wasseranteils (80° C) eingerührt (Magnetrührer), der restliche Wasseranteil (20° C) dazugegeben und so lange gerührt bis die HPMC vollständig gelöst war. Nach Zugabe des pharmazeutischen Wirkstoffes wurde die Lösung unter ständigem Rühren auf 50 bis 60° C erhitzt.

Tabelle 3 zeigt die Zusammensetzung der Wirkstoffbeschichtungslösung.

**Tabelle 3:**

| **Substanz** | **Hersteller** | **Bezeichnung** | **Gehalt [%]** |
|---|---|---|---|
| Diclofenac-Na | Sigma-Aldrich, DE | Pharm. Wirkstoff | 5,0 |
| HPMC Pharmacoat 606 | Shin-Etsu Chemical, JP | Bindemittel | 2,0 |
| Demin. Wasser | - | Lösungsmittel | 93,0 |

Die Temperatur der Wirkstoffbeschichtungslösung betrug 50 bis 60° C. 215 g der Wirkstoffbeschichtungslösung wurden verwendet, was bei einer Wirkstoffkonzentration von 5 % eine Menge von 25 mg Wirkstoff pro 500 mg beladenen Pellets ergibt. Die Beschichtung wurde in einem Wirbelschichttrockner mit Bodensprühsystem (Aeromatic Strea, Aeromatic, Zweistoffdüse mit 1,2 mm Durchmesser) durchgeführt. Tabelle 4 zeigt die Beschichtungsparameter.

**Tabelle 4:**

| | |
|---|---|
| Batchgröße | 200 g |
| Betriebsart | Bodensprühsystem |
| Zuluft | 80 m³/h |
| Sprühdruck | 1 bar |
| Sprührate | ca. 3 ml/min |
| Zulufttemperatur | 57° C |
| Ablufttemperatur | 51 - 53° C |

### Beispiel 3: Beschichtung der wirkstoffbeladenen Pellets mit einem Film zur kontrollierten Freisetzung des Wirkstoffs

Die Beschichtung wurde ebenfalls mit dem Wirbelschichttrockner mit Bodensprühsystem (Aeromatic Strea, Fa. Aeromatic) durchgeführt.

Die Pellets wurden jeweils so lange mit der Beschichtungsdispersion beschichtet bis eine 5 bzw. 10 %ige Gewichtszunahme (bezogen auf die ursprüngliche Pelletmasse) erreicht wurde.

Tabelle 5 zeigt die Zusammensetzung der Beschichtungsdispersion.

**Tabelle 5:**

| **Substanz** | **Bezeichnung** | **Hersteller** | **Gehalt [%]** |
|---|---|---|---|
| Eudragit® RS 30 D | Filmbildendes Polymer | Degussa, DE | 33,30 |
| Talk | Gleitmittel | Sigma-Aldrich, DE | 7,50 |
| Triethylcitrat | Verflüssiger | Fluka Chemie, CH | 2,00 |
| Demin. Wasser | Lösungsmittel | - | 57,20 |

Feststoffgehalt der Beschichtungsdispersion: 19,56 %.

### Herstellung der Dispersion

Wasser, Talk und Triethylcitrat wurden in einem Zwangsmischer vermischt. Die erhaltene Dispersion wurde unter vorsichtigem Rühren mit einem Magnetrührer zu der Eudragit® (Fa. Degussa, DE) Dispersion gegeben und anschließend durch ein 0,5 mm Sieb filtriert.

Tabelle 6 zeigt die Prozessparameter für die Beschichtung der Pellets.

**Tabelle 6:**

| | |
|---|---|
| Batchgröße | 150 g |
| Betriebsart | Bodensprühsystem |
| Zuluft | 80 m³/h |
| Sprühdruck | 0,8 bar |
| Sprührate | 3 - 5 ml/min |
| Zulufttemperatur | 48 - 51° C |
| Ablufttemperatur | 37 - 43° C |

Theoretischer Gehalt an Beschichtungsdispersion: 186 g, berechnet für 10 % Trockenpolymergehalt.

### Beispiel 4: Wirkstofffreisetzung aus den erfindungsgemäßen Pellets

Starterpellets aus MCC, purem Isomalt und der erfindungsgemäßen Zusammensetzung wurden wie in den Beispielen 2 und 3 beschrieben mit Wirkstoff (Diclofenac-Na) beladen und mit Eudragit® RS beschichtet bis eine 5 bzw. 10 %ige Gewichtszunahme (bezogen auf die ursprüngliche Pelletmasse) erreicht wurde. Die Freisetzung des Wirkstoffs wurde in einem Freisetzungstester durchgeführt, beschrieben in USP 30 dissolution apparatus I (Basket-Methode; Hanson SR8-Plus™ Dissolution Test Station mit E-Sonde; AutoPlus Maximizer und Multifill Collector, Fa. Hanson Research, USA). Das Volumen (900 ml) des Freisetzungsmediums (Phosphatpufferlösung pH-Wert = 6,8 +/- 0,05) wurde in den Gefäßen bei einer Temperatur von 37° C +/- 0,5° C gehalten. Gerührt wurde mit 100 rpm und die Wirkstoffkonzentration einer durch einen 10 µm Filter filtrierten Probe mittels UV-Spektroskopie bei 276 nm Wellenlänge gemessen (Agilent 8453 UV/VIS Spektrophotometer, Fa. Agilent Technologies).

Figur 3 zeigt die Freisetzung des Wirkstoffs aus Pellets aus der erfindungsgemäßen Zusammensetzung (Verhältnis Isomalt zu MCC: 50:50, 30:70 und 10:90 Gew.-% TS), die mit jeweils 5 und 10 % Eudragit® RS beschichtet wurden. Es ist deutlich zu erkennen, dass die erfindungsgemäßen Starterpellets trotz des hohen Gehaltes an MCC den Wirkstoff schnell und vollständig freisetzen.

### Beispiel 5: Wirkstofffreisetzung aus verschiedenen Pellets bei unterschiedlicher Osmolalität

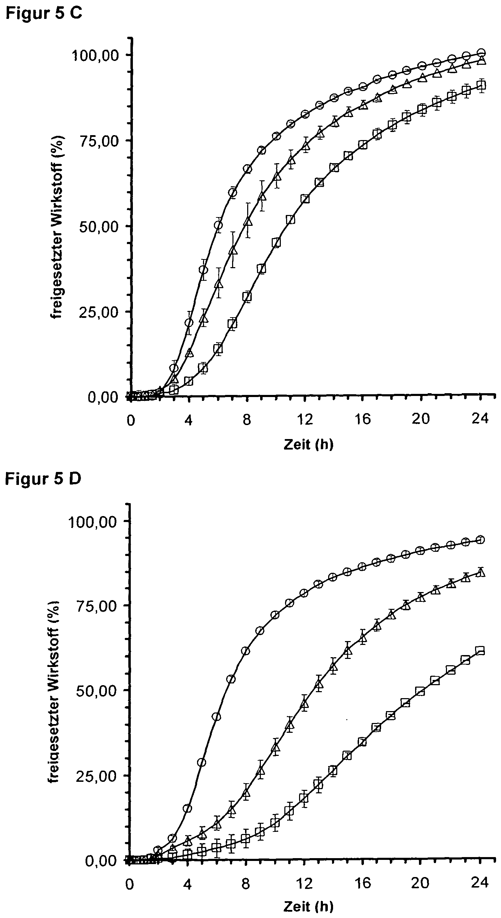

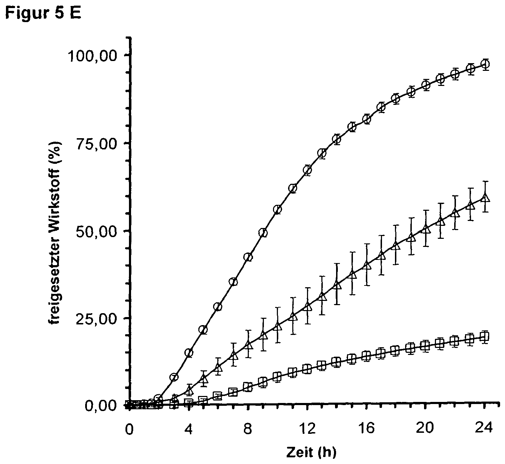

Die Freisetzung des Wirkstoffs Diclofenac-Na wurde wie in Beispiel 4 beschrieben bei Pellets aus purem Isomalt (Figur 4 und 5A), MCC (Figuren 4 und 5B) und dem erfindungsgemäßen Gemisch (50:50 Figuren 4 und 5 C; 30:70 Figuren 4 und 5 D; 10:90 Figuren 4 und 5E) mit 5 bzw. 10 % Eudragit® RS Beschichtung untersucht (Figur 4, 5 % Beschichtung; Figur 5, 10 % Beschichtung). Hierbei wurde zunächst die Freisetzung unter normalen Bedingungen (normale Osmolalität, 0,106 Osm/kg) untersucht (siehe Figuren 4 und 5 jeweils Linie mit Kreis). Die Osmolalität wurde dann im Freisetzungsmedium durch Zugabe von 0,25 mol/l Glukoseanhydrat (siehe Figuren 4 und 5 Linie mit Dreieck) bzw. 0,5 mol/l Glukoseanhydrat (siehe Figuren 4 und 5 Linie mit Quadrat) erhöht. Die Osmolalität wurde mit einem Osmometer (Knauer-OSMO, Modell 2320) mittels Gefrierpunkterniedrigung bestimmt. Nach Zugabe von 0,25 mol/l bzw. 0,5 mol/l Glukoseanhydrat wurde eine Osmolalität von 0,483 Osm/kg bzw. 0,706 Osm/kg gemessen.

Figur 4 zeigt deutlich, dass bei einem Beschichtungsgrad von 5 % die Freisetzungskinetik des Wirkstoffs aus MCC Pellets im Vergleich mit den erfindungsgemäßen Pellets oder auch purem Isomalt sehr viel langsamer ist (Figuren 4A bis E, Linien mit Kreis) und mit Erhöhung der Osmolalität des Mediums stark beeinflusst wird, wohingegen die Pellets aus der erfindungsgemäßen Zusammensetzung und purem Isomalt in entscheidend geringerem Maße von der erhöhten Osmolalität beeinflusst werden (Figur 4A bis E, Linien mit Dreieck).

Auch bei einer weiteren Erhöhung der Osmolalität durch 0,5 mol/l Glukoseanhydrat sind die erfindungsgemäßen Pellets weniger stark beeinflusst als MCC Pellets, d. h. der Wirkstoff wird schneller freigesetzt (Figuren 4A bis E, Linien mit Quadrat).

Figur 5 zeigt deutlich, dass auch bei einem Beschichtungsgrad von 10% die Freisetzungskinetik des Wirkstoffs aus MCC Pellets im Vergleich mit den erfindungsgemäßen Pellets deutlich langsamer ist (Figur 5A bis E, Linien mit Kreis) und mit Erhöhung der Osmolalität des Mediums annähernd zum Erliegen kommt, wohingegen die Pellets aus der erfindungsgemäßen Zusammensetzung und reinem Isomalt in entscheidend geringerem Maße von der erhöhten Osmolalität beeinflusst werden (Figur 5A bis E, Linien mit Dreieck). Auch bei einer weiteren Erhöhung der Osmolalität durch 0,5 mol/l Glukoseanhydrat sind die erfindungsgemäßen Pellets weniger stark beeinflusst als MCC Pellets, d. h. der Wirkstoff wird schneller freigesetzt (Figur 5A bis E, Linien mit Quadrat).

### Beispiel 6: Untersuchung der Löslichkeit verschiedener Pellets

Zur Untersuchung der Löslichkeit von Starterpellets wurden bekannte Saccharose basierte Starterpellets und Pellets aus purem Isomalt mit den erfindungsgemäßen Starterpellets verglichen. Hierzu wurden jeweils einzelne Pellets in entionisiertes Wasser (20° C) gegeben und die Zeit bis zur vollständigen Auflösung oder zum vollständigen Zerfall gemessen. Tabelle 1 zeigt die Ergebnisse für die untersuchten Starterpellets.

**Tabelle 1**

| **Starterpellet** | **Zerfall / Lösung** | **Zeit** |
|---|---|---|
| Saccharose | Zerfall und Lösung | 180 sec |
| Isomalt | Lösung | 135 - 180 sec |
| MCC | - | > 15 min |
| Erfindungsgemäße Zusammensetzung (50:50; 30:70; 10:90) | - | > 15 min |

Tabelle 1 zeigt deutlich, dass sich Pellets aus Saccharose oder Isomalt sehr schnell auflösen bzw. zerfallen. Dies ist während der Verarbeitung in der Wirbelschicht, vor allem bei der Beschichtung mit Wirkstoff oder einem Überzug, nachteilig, da aufgrund der Auflösungserscheinungen nur geringere Sprühraten eingesetzt werden können. Dies führt zu verlängerten Verweilzeiten und zu einem höheren Verbrauch von Beschichtungsmaterial. Ein weiterer Nachteil dieses Auflösungsverhaltens zeigt sich nach der Einnahme eines mit Wirkstoff und Polymerüberzug beschichteten Pellets aus Saccharose oder purem Isomalt. Durch die leichte Löslichkeit bildet sich zwischen dem Polymerüberzug und dem eigentlichen Kern eine gesättigte Lösung oder Suspension des Wirkstoffs was zu einer Verringerung der mechanischen Widerstandsfähigkeit des Polymerüberzugs führt. Durch die Bewegung des Gastrointestinalen Trakts besteht dann die Gefahr, dass der Polymerüberzug mechanisch beschädigt wird. Da die Polymerhülle zur Kontrolle der Wirkstofffreisetzung durch Auflösung oder Porenbildung dient, kann als Folge der mechanischen Beschädigung ein unerwünschtes Freisetzungsprofil des Wirkstoffs auftreten. Die erfindungsgemäßen Starterpellets zeigen hier vorteilhafterweise ein den MCC-Pellets ähnliches Verhalten, das zu einer besseren Verarbeitbarkeit führt.

## Patentansprüche

1. Pharmazeutisches Starterpellet aus einer Zusammensetzung, enthaltend
a) ein Gemisch von 6-O-α-D-Glucopyranosyl-D-Sorbit (1,6-GPS) und 1-O-α-D-Glucopyranosyl-D-Mannit (1,1-GPM) und außerdem
b) mikrokristalline Cellulose (MCC).

2. Pharmazeutisches Starterpellet gemäß Anspruch 1, wobei Komponente a) ein Gemisch von 47 bis 53 Gew.-% 1,6-GPS und 53 bis 47 Gew.-% 1,1-GPM (bezogen auf die Trockensubstanz) ist.

3. Pharmazeutisches Starterpellet gemäß Anspruch 1, wobei Komponente a) ein Gemisch von 20 bis 48 Gew.-% 1,6-GPS und 80 bis 52 Gew.-% 1,1-GPM (bezogen auf die Trockensubstanz) ist.

4. Pharmazeutisches Starterpellet gemäß einem der vorstehenden Ansprüche, wobei die Komponenten a) und b) in der Zusammensetzung in einem Verhältnis von 1:99 bis 99:1 Gew.-% (bezogen auf die Trockensubstanz) enthalten sind.

5. Pharmazeutisches Starterpellet gemäß einem der Ansprüche 1 bis 3, wobei die Komponenten a) und b) in der Zusammensetzung in einem Verhältnis von 5:95 bis 60:40 Gew.-% (bezogen auf die Trockensubstanz) enthalten sind.

6. Pharmazeutisches Starterpellet gemäß einem der Ansprüche 1 bis 3, wobei die Komponenten a) und b) in der Zusammensetzung in einem Verhältnis von 10:90 bis 50:50 Gew.-% (bezogen auf die Trockensubstanz) enthalten sind.

7. Pharmazeutisches Starterpellet gemäß einem der vorstehenden Ansprüche, wobei das pharmazeutische Starterpellet einen Durchmesser von 0,1 bis 1,5 mm aufweist.

8. Pharmazeutisches Starterpellet gemäß einem der vorstehenden Ansprüche, wobei das pharmazeutische Starterpellet mit mindestens einem pharmazeutischen Wirkstoff beschichtet ist.

9. Pharmazeutisches Starterpellet gemäß einem der vorstehenden Ansprüche, wobei das pharmazeutische Starterpellet mindestens einen pharmazeutischen Wirkstoff in der Zusammensetzung enthält.

10. Pharmazeutisches Starterpellet gemäß dem vorstehenden Anspruch 8 oder 9, wobei das pharmazeutische Starterpellet eine äußere Schicht aufweist, die speziell zur kontrollierten Freisetzung eines pharmazeutischen Wirkstoffs ausgebildet ist.

11. Verwendung der in einem der Ansprüche 1 bis 6 charakterisierten Zusammensetzung als Bestandteil eines pharmazeutischen Starterpellets zur Verbesserung der Freisetzung eines pharmazeutischen Wirkstoffs von dem Pellet.

12. Verwendung gemäß Anspruch 11, wobei die verbesserte Freisetzung des pharmazeutischen Wirkstoffs weitgehend unabhängig von der Osmolalität des Umgebungsmediums ist.

13. Verwendung der in einem der Ansprüche 1 bis 6 charakterisierten Zusammensetzung als Bestandteil eines pharmazeutischen Starterpellets zur Verringerung des Abriebs während der Verarbeitung.

14. Verwendung der in einem der Ansprüche 1 bis 6 charakterisierten Zusammensetzung als Bestandteil eines pharmazeutischen Starterpellets zur Verbesserung der kontrollierten Beschichtung des Starterpellets mit einem pharmazeutischen Wirkstoff.

## Claims

1. Pharmaceutical starter pellet consisting of a composition containing:
a) a mixture of 6-O-α-D-glucopyranosyl-D-sorbitol (1,6-GPS) and 1-O-α-D-glucopyranosyl-D-mannitol (1,1-GPM) and also
b) microcrystalline cellulose (MCC).

2. Pharmaceutical starter pellet according to claim 1, wherein component a) is a mixture of 47 to 53 % by weight 1,6-GPS and 53 to 47 % by weight 1,1-GPM (based on the dry matter).

3. Pharmaceutical starter pellet according to claim 1, wherein component a) is a mixture of 20 to 48 % by weight 1,6-GPS and 80 to 52 % by weight 1,1-GPM (based on the dry matter).

4. Pharmaceutical starter pellet according to any of the preceding claims, wherein the components a) and b) are contained in the composition in a ratio of 1:99 to 99:1 % by weight (based on the dry matter).

5. Pharmaceutical starter pellet according to any of claims 1 to 3, wherein the components a) and b) are contained in the composition in a ratio of 5:95 to 60:40 % by weight (based on the dry matter).

6. Pharmaceutical starter pellet according to any of claims 1 to 3, wherein components a) and b) are contained in the composition in a ratio of 10:90 to 50:50 % by weight (based on the dry matter).

7. Pharmaceutical starter pellet according to any of the preceding claims, wherein the pharmaceutical starter pellet has a diameter of 0.1 to 1.5 mm.

8. Pharmaceutical starter pellet according to any of the preceding claims, wherein the pharmaceutical starter pellet is coated with at least one pharmaceutical active ingredient.

9. Pharmaceutical starter pellet according to any of the preceding claims, wherein the pharmaceutical starter pellet contains at least one pharmaceutical active ingredient in the composition.

10. Pharmaceutical starter pellet according to any of preceding claims 8 and 9, wherein the pharmaceutical starter pellet has an outer layer which is formed especially for controlled release of a pharmaceutical active ingredient.

11. Use of the composition **characterised in** any of claims 1 to 6 as a component of a pharmaceutical starter pellet for improving the release of a pharmaceutical active ingredient from the pellet.

12. Use according to claim 11, wherein the improved release of the pharmaceutical active ingredient is largely independent of the osmolality of the ambient medium.

13. Use of the composition **characterised in** any of claims 1 to 6 as a component of a pharmaceutical starter pellet for reducing abrasion during processing.

14. Use of the composition **characterised in** any of claims 1 to 6 as a component of a pharmaceutical starter pellet for improving the controlled coating of the starter pellet with a pharmaceutical active ingredient.

## Revendications

1. Pellet de départ pharmaceutique à base d'une composition, contenant :
a) un mélange de 6-O-α-D-glucopyranosyl-D-sorbitol (1,6-GPS) et de 1-O-α-D-glucopyranosyl-D-mannitol (1,1-GPM) et, en outre
b) de la cellulose microcristalline (MCC).

2. Pellet de départ pharmaceutique selon la revendication 1, dans lequel la composante a) est un mélange de 47 à 53 % en poids de 1,6-GPS et de 53 à 47 % en poids de 1,1-GPM (par rapport à la substance sèche).

3. Pellet de départ pharmaceutique selon la revendication 1, dans lequel la composante a) est un mélange de 20 à 48 % en poids de 1,6-GPS et de 80 à 52 % en poids de 1,1-GPM (par rapport à la substance sèche).

4. Pellet de départ pharmaceutique selon l'une des revendications précédentes, dans lequel les composantes a) et b) dans la composition sont contenues dans un rapport de 1 : 99 à 99 : 1 en % en poids (par rapport à la substance sèche).

5. Pellet de départ pharmaceutique selon l'une des revendications 1 à 3, dans lequel les composantes a) et b) dans la composition sont contenues dans un rapport de 5 : 95 à 60 : 40 en % en poids (par rapport à la substance sèche).

6. Pellet de départ pharmaceutique selon l'une des revendications 1 à 3, dans lequel les composantes a) et b) dans la composition sont contenues dans un rapport de 10 : 90 à 50 : 50 en % en poids (par rapport à la substance sèche).

7. Pellet de départ pharmaceutique selon l'une des revendications précédentes, dans lequel le pellet de départ pharmaceutique présente un diamètre de 0,1 à 1,5 mm.

8. Pellet de départ pharmaceutique selon l'une des revendications précédentes, dans lequel le pellet de départ pharmaceutique est revêtu d'au moins une matière active pharmaceutique.

9. Pellet de départ pharmaceutique selon l'une des revendications précédentes, dans lequel le pellet de départ pharmaceutique contient au moins une matière active pharmaceutique dans la composition.

10. Pellet de départ pharmaceutique selon l'une des revendications précédentes 8 ou 9, dans lequel le pellet de départ pharmaceutique présente une couche extérieure qui est spécialement conçue pour la libération contrôlée d'une matière active pharmaceutique.

11. Utilisation de la composition **caractérisée** dans l'une des revendications 1 à 6 en tant que composant d'un pellet de départ pharmaceutique pour l'amélioration de la libération d'une matière active pharmaceutique du pellet.

12. Utilisation selon la revendication 11, dans laquelle la libération améliorée de la matière active pharmaceutique est largement indépendante de l'osmolalité du milieu environnant.

13. Utilisation de la composition **caractérisée** dans l'une des revendications 1 à 6 en tant que composant d'un pellet de départ pharmaceutique destiné à la diminution de l'abrasion au cours de la fabrication.

14. Utilisation de la composition **caractérisée** dans l'une des revendications 1 à 6 en tant que composant d'un pellet de départ pharmaceutique destiné à l'amélioration du revêtement contrôlé du pellet de départ avec une matière active pharmaceutique.
